# EUROPEAN PATENT SPECIFICATION

(11) **EP 1 061 825 B1**
(45) Date of publication and mention of the grant of the patent: **30.07.2003**
(21) Application number: 99913547.8
(22) Date of filing: 02.03.1999
(51) Int. Cl.: A44B 18/00, A61F 13/62

(54) **TOUCH FASTENER TAPE**
FLÄCHENHAFTVERSCHLUSSBAND
BANDE DE FERMETURE PAR CONTACT

(30) Priority: 02.03.1998 US 32983
(43) Date of publication of application: 27.12.2000
(73) Proprietor: VELCRO INDUSTRIES B.V., Curacao (AN)
(72) Inventor: COSLOVI, Giuliano, I-20052 Monza (IT); CLERICI, Piero, Rusconi, I-20121 Milano (IT)
(74) Representative: Müller-Boré & Partner Patentanwälte
(86) International application number: PCT/IB99/00758
(87) International publication number: WO 99/044457

(56) References cited:
- EP-A- 0 330 415
- EP-A- 0 429 249
- EP-A- 0 568 919
- EP-A- 0 694 642
- WO-A-96/03101
- US-A- 4 705 710
- US-A- 4 714 096

## Description

This invention relates to touch fastener tapes, such as for hook-and-loop fastening, and methods for producing such tapes. Such a tape and method is known from e.g. US-A-4 705 710.

Textile based touch fasteners tapes, i.e. tapes of woven or knit fabric, are often used in the weft, or cross machine, direction. These tapes are cut from the textile to have the lengthwise direction of the tape oriented with the weft or cross-machine direction of the textile fabric. Thus tension on the tape occurs in the weft or cross-machine direction of the textile fabric.

It is desirable for touch fasteners to exhibit elasticity and to form secure fastenings for making secure closures.

### Summary of the Invention

The invention features a textile fastener tape having its tension direction oriented with the weft direction of the textile fabric, one or more sections of the tape being elastically stretchable and one or more sections being not stretchable as a result of the construction of the textile fabric itself.

In preferred embodiments the fastener tape is produced on standard weaving machines, using warp and weft yarns in which selected warp yarns are elastic and are provided in selected localized patterns in which sections of the yarns have a component of their extent in the cross machine or weft direction. Alternatively, the fastener tape is produced on knitting machines using elastic warp yarns.

The resulting tapes are stretchy in the weft direction only in the sections where the elastic yearns have extent in the weft direction, other sections of the tape being non stretchable.

In a general aspect of the invention, the fastener tape includes a textile backing defining a warp and a weft direction and, in the weft direction, having a first region and second region. The first region is substantially non-stretchable in the weft direction and the second region is substantially elastically stretchable in the weft direction. In an array of fastener elements extends from a selected region of the tape.

The region of the fastener tape that is substantially elastically stretchable observably increases in length in response to a typical force applied to the fastener tape and, upon removal of the force, retracts to approximately the original length of the region.

A region of the fastener tape that is substantially non-stretchable does not observably increase in length in response to typical forces applied to a fastener tape.

Important embodiments of this aspect of the invention include one or more of the following features.

The fastener tape includes a region having fastener elements, the region being substantially non-stretchable in the weft direction, or the fastener tape includes a region having no fastener elements and which is substantially non-stretchable in the weft direction, e.g. suitable for securing to an object or the fastener tape includes at least two non-stretchable regions, one having fastener elements and one being disposed to be permanently secured to an object such as a diaper.

An elastic region of the fastener tape includes a warp pattern defined by inelastic warp yarns and elastic warp yarns having a portion extending in a weft direction for crossing at least one inelastic warp yarn. Many of the elastic warp yarns each cross more than one inelastic warp yarn. The textile backing is woven. The textile backing is a leno weave.

The textile backing is a knit.

The fastener tape is in the form of a fastener tab having a portion secured to an object and a portion protruding beyond the object having the second region with the fastener elements spaced from the object by the second region. The object is a garment (e.g. a diaper).

The fastener tape is formed by first forming a textile fabric having a weft direction by knitting or wearing, the fabric having bands extending in the weft direction, at least one of the bands being stretchable in the weft direction, and one of the band not being stretchable, and then forming the tape from a portion of the fabric, the tape extending in the weft direction of the fabric and including portions of a stretchable and a non-stretchable band.

Other features and advantages will be apparent from the following description and claims.

### Brief Description of the Drawings

Fig. 1 is a plan view of a fastener tape.
Fig. 2 is a schematic illustration of a fabric from which the fastener tape is cut.
Fig. 3 is a side view of the fastener tape of Fig. 1.
Fig. 4 illustrates the structure of an elastic section of a woven fastener tape.
Figs. 5A and 5B show the elastic section structure of a woven fastener tape under tension and relaxed, respectively.
Fig. 6 illustrates the structure of an elastic section of a knit.
Fig. 7 shows the fastener tape on a diaper.

### Description

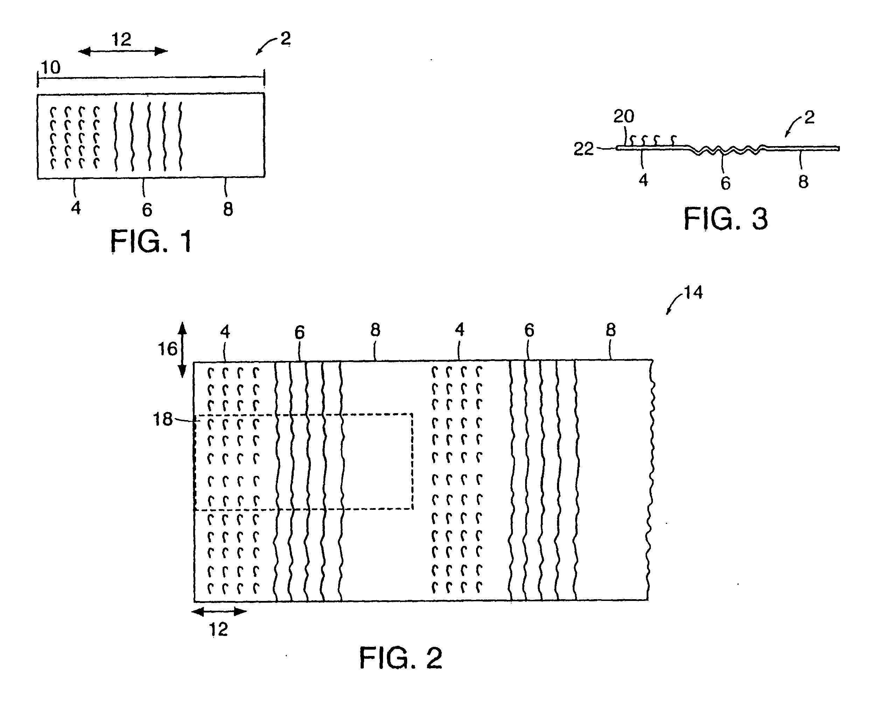

Referring to Figs. 1 and 2, a textile fastener tape 2 includes a fastener section 4, an elastic section 6, and a tab section 8 arranged sequentially across a length 10 extending in weft direction 12 of the textile fabric 14 from which the tape is cut. Sections 4, 6, 8 exhibit different elastic behavior in weft direction 12, being, non stretchable, stretchable, and non stretchable, respectively, as a result of the pattern of construction of the textile fabric. In response to a typical applied force along weft direction 12 of the fastener tape, fastener section 4 and tab section 8 do not substantially stretch while elastic section 6 observably stretches.

Referring to Fig. 2, fastener tape 2 (Fig. 1) is produced from fabric 14 which includes multiple fastener sections 4, elastic sections 6, and tab sections 8 that extend as continuous bands in warp direction 16 of the fabric, as it is formed. A portion of fabric 16 as indicated by dotted line 18 is cut to form the fastener tape 2 of Fig. 1, the lengthwise direction 12 of the tape extending in the weft direction 12 of the textile fabric.

Referring to Fig. 3, an array of male fastener elements 20 extends from a base fabric 22 of fastener tape 2 for the purpose of engaging cooperating fastener elements to make a closure. Male fastener elements 20 can be hook-shaped, as shown, or mushroom-shaped.

In either case, the fastener elements can be formed by cutting loops of the formed textile by techniques well-known in the touch fastener art. For instance loops of monofilament yarns have portions cut away to form loop-engageable fastener hooks or ends of cut loops of monofilament are deformed by heat to form loop-engeagable mushroom heads. In other cases multiple yarn loops form hook-engageable fastener loops.

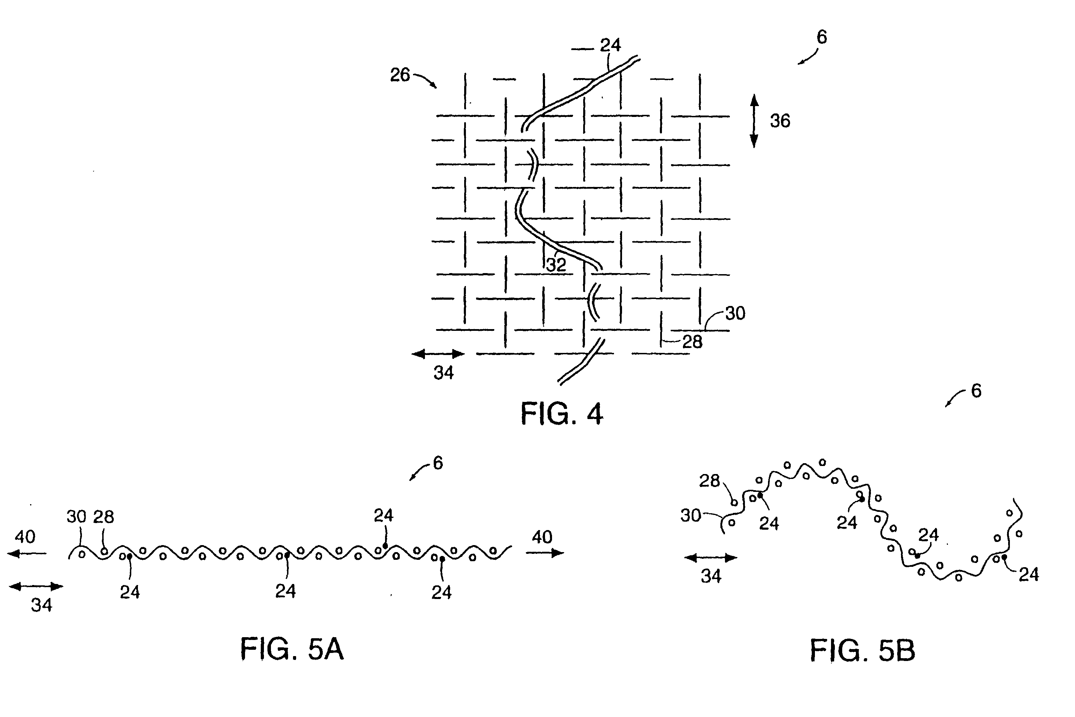

Referring to Fig. 4, elastic section 6 is manufactured by incorporating a number of elastic warp yarns 24 into a woven fabric 26 being woven primarily of inelastic warp yarns 28 and in elastic weft yarns 30.
The selected weaving pattern causes elastic warp yarns 24 to have weft direction components of extension in section 6, see Fig. 2. Here, localized sections 32 of warp yarns extend in weft direction 34, though the overall direction of extent of these yarns is in warp direction 36. As in a leno weave, elastic warp yarns 24 cross adjacent inelastic warp yarns 32. In some cases, individual elastic warp yarns 24 cross over multiple inelastic warp yarns 28.

Examples of elastic warp yarn 24 include elastomeric yarn (110 decitex), either single-component or coated, and lycra (235 decitex) coated with nylon. Elastic section 6 is formed to have a density of approximately seventeen elastic warp yarns 24 per one inch width fabric. In some cases, it is desirable to produce woven fabric 26 having multiple elastic sections 6 with different elastic properties (e.g. different elongations).

Referring to Fig. 5A, a cross-section of a portion of elastic section 6 is placed under tension by a force, indicated by arrows 40, in weft direction 34. The force, for example, is applied during the weaving process used to manufacture fastener tape 2 and causes elastic warp yarns 24 to stretch in weft direction 34 while being incorporated into fabric 26 (Fig. 4). Alternatively, the force can be applied to fastener tape 2 in use. Elastic warp yarns 24 elastically elongate in response to the force.

Referring to Fig. 5B, when the force is removed elastic warp yarns 24 contract and elastic section 6 returns to an unstretched state. Woven fabric 26, in the region where the elastic yarns are incorporated in the weave pattern forms a wave-like structure as the inelastic warp yarns 32, crossed by elastic warp yarns 24 (Fig. 4), are pulled in by the contraction.

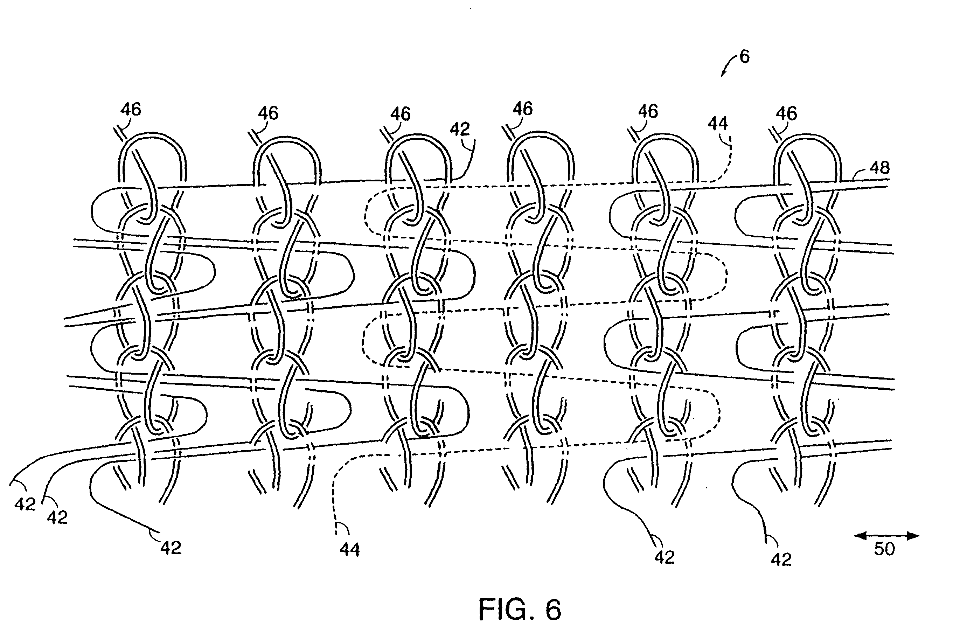

Referring to Fig. 6, in a knitted construction following the same principles, section 6 of a knitted fabric 48 includes a series of wales 46, that is rows of loops of inelastic yarns that extend across knitted fabric 48, in between which elastic weft yarns 44 and inelastic weft yarns 42 are knitted in respective sections of the textile fabric corresponding to sections 6 in FIGS 1 and 2. Elastic weft yarns 44 and inelastic weft yarns are knitted in between two non-adjacent wales 46 such that at least one wale 46 is common to both elastic weft yarn 44 and inelastic warp yarn 42. Because of the extensibility in weft direction 50 of elastic weft yarns 44, elastic section 6 elastically stretches in response to a force applied to knitted fabric 48 in weft direction 50, as described with respect to woven fabric .

Other knitting patterns having different types of interlacing between elastic yarns 44 and inelastic yarns 42 may be employed to effect the elasticity of elastic section 6.

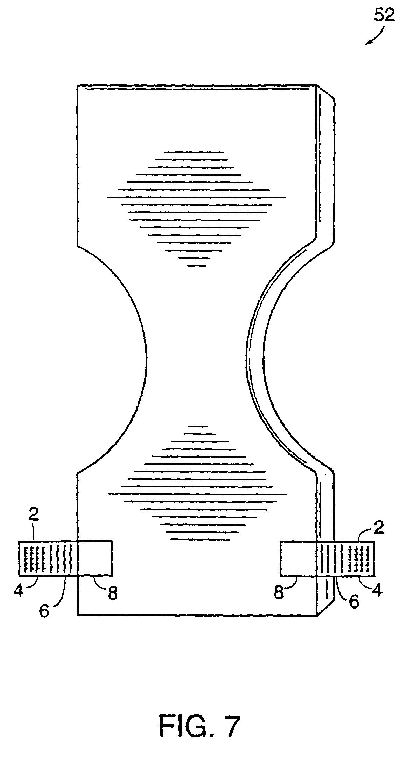

Referring to Figs. 1 and 7, fastener tape 2 is permanently adhered at its tab section 8 to a garment and used to secure the garment, in this case, diaper 52, around a human body. In use, fastener tape 2 is pulled in the weft direction 12 to engage male fastener elements 20 (Fig. 3) of fastener section 4 with female fastener elements (not shown) on an opposite side of garment 52. As fastener tape 2 is pulled, elastic section 6 stretches in the weft direction while fastener section 4 and tab section 8 do not stretch. The non-stretchability of the respective sections 4 and 8, contribute to the performance of the fasteners of section 4 and the strength of the bond of section 8 to the garment. After fastener elements 14 are correspondingly engaged, the tendency for elastic section 6 to return to its unstretched state provides a force that secures diaper 52 around the body and helps to maintain the engagement of the closure.

Other features and embodiments are also within the scope of the following claims.

## Claims

1. A fastener tape (2) comprising:
a textile backing formed by a pattern of selected yarns defining a warp direction and a weft direction, **characterised in that** the textile backing (14), as a result of the pattern of selected yarns, having, in the weft direction (12), a first region (4 or 8) that is substantially non-stretchable in the weft direction and a second region (6 )that is substantially elastically stretchable in the weft direction.

2. The fastener tape of claim 1 in which
an array of fastener elements (20) extend from the first region (4).

3. The fastener tape of claim 1 or 2 having fastener elements in the form of hook-engageable hooks (20).

4. The fastener tape of any of the foregoing claims wherein the textile backing includes at least two non-stretchable in the weft direction, regions (4/6), one of which carrying fastener elements and one of which having no fastener elements.

5. The fastener tape of claim 4 in which one of the non-stretchable regions (4) carries an array of fastener elements and the other non-stretchable region comprises a tab arranged to be joined to an article, e.g., an article of clothing such as a diaper.

6. The fastener tape of any of the foregoing claims wherein the elastically stretchable region includes a warp pattern defined by inelastic warp yarns and elastic warp yarns, the elastic warp yarns each having a portion extending in a weft direction for crossing at least one of the inelastic warp yarns.

7. The fastener tape of claim 6 wherein the textile backing is woven and comprises a leno weave.

8. The fastener tape of claims 6 or 7 wherein many of the elastic warp yarns each cross more than one inelastic warp yarn.

9. The fastener tape of any of the claims 1-6 wherein the textile backing comprises a knit.

10. The fastener tape of any of the foregoing claims in the form of a fastener tab having a portion secured to an object and a portion protruding beyond the object, the protruding portion having a stretchable region and a second region from which fastener elements extend, the second region being spaced from the object by said stretchable region.

11. The fastener tape of claim 10 wherein the object comprises a garment.

12. The fastener tape of claim 11 wherein the garment comprises a diaper.

13. A method of forming the fastener tape of any of the foregoing claims **characterized in** forming a textile fabric (14) having a weft direction (12) by knitting or weaving, the fabric having bands extending in the warp direction (16), at least one of the bands (6) being stretchable in the weft direction, and one of the bands (4 or 8) being non-stretchable, and forming the tape from a portion (18)of the fabric, the tape extending in the weft direction of the fabric and including portions of a stretchable and a non-stretchable band.

14. The method of claim 13 further **characterized in** forming an array of fastener elements (20) in one of said non-stretchable bands (4 or 8).

15. The method of claim 13 further **characterized in** forming in the stretchable bands (6) a warp pattern defined by inelastic warp yarns and elastic warp yarns, the elastic warp yarns each having a portion extending in the weft direction for crossing at least one of the inelastic warp yarns.

16. The method of claim 15 **characterized in that** the elastic warp yarns each cross more than one inelastic warp yarn.

17. The method of claim 13 further **characterized in that** the fastener tape has a non-stretchable portion secured to an object and a portion protruding beyond the object, the protruding portion having a stretchable region and a non-stretchable region from which fastener elements extend, the non-stretchable region being spaced from the object by the stretchable region.

## Patentansprüche

1. Festlegungsband bzw. Verschlußband (2) umfassend:
einen textilen Rücken, der aus einem Muster von gewählten Garnen gebildet ist, die eine Kettfadenrichtung und eine Schußfadenrichtung definieren, **dadurch gekennzeichnet, daß** der textile Rücken (14) als ein Ergebnis des Musters der gewählten Garne in der Schußfadenrichtung (12) einen ersten Bereich (4 oder 8), welcher im wesentlichen nicht dehnbar in der Schußfadenrichtung ist, und einen zweiten Bereich (6) aufweist, der im wesentlichen elastisch dehnbar in der Schußfadenrichtung ist.

2. Festlegungsband nach Anspruch 1, in welchem sich ein Feld bzw. eine Anordnung von Festlegungselementen (20) sich von dem ersten Bereich (4) erstreckt.

3. Festlegungsband nach Anspruch 1 oder 2, welches Festlegungselemente in der Form von in Haken eingreifbaren Haken (20) aufweist.

4. Festlegungsband nach einem der vorhergehenden Ansprüche, worin der textile Rücken wenigstens zwei in der Schußfadenrichtung nicht dehnbare Bereiche (4, 8) aufweist, von welchen einer Festlegungselemente trägt und einer keine Festlegungselemente trägt.

5. Festlegungsband nach Anspruch 4, in welchem einer der nicht dehnbaren Bereiche (4) ein Feld bzw. eine Anordnung von Festlegungselementen trägt und der andere nicht dehnbare Bereich einen Fortsatz bzw. eine Lasche umfaßt, der angeordnet ist, um mit einem Gegenstand, z.B. einem Kleidungsstück, wie einer Windel, verbunden zu werden.

6. Festlegungsband nach einem der vorhergehenden Ansprüche, worin der elastisch dehnbare Bereich ein Kettfadenmuster umfaßt, das durch nicht elastische Kettfäden und elastische Kettfäden definiert ist, wobei die elastischen Kettfäden jeweils einen Bereich aufweisen, der sich in einer Schußfadenrichtung zum Kreuzen mit wenigstens einem der nicht elastischen Kettfäden erstreckt.

7. Festlegungsband nach Anspruch 6, worin der textile Rücken gewebt ist und ein Gaze-Gewebe bzw. ein Drehergewebe-Bindungsmuster umfaßt.

8. Festlegungsband nach Anspruch 6 oder 7, worin zahlreiche der elastischen Kettfadengarne jeweils mehr als ein nicht elastisches Kettfadengarn kreuzen.

9. Festlegungsband nach einem der Ansprüche 1 bis 6, worin der textile Rücken ein Gewirk bzw. Strick- bzw. Maschenware umfaßt.

10. Festlegungsband nach einem der vorhergehenden Ansprüche in der Form eines Festlegungsfortsatzes bzw. -lasche, der einen Bereich an einem Gegenstand festgelegt und einen Bereich über den Gegenstand vorragend aufweist, wobei der vorragende Bereich einen dehnbaren Bereich und einen zweiten Bereich aufweist, von welchem sich die Festlegungselemente erstrecken, wobei der zweite Bereich von dem Gegenstand durch den dehnbaren Bereich beabstandet ist.

11. Festlegungsband nach Anspruch 10, worin der Gegenstand ein Kleidungsstück umfaßt.

12. Festlegungsband nach Anspruch 11, worin das Kleidungsstück eine Windel umfaßt.

13. Verfahren zum Ausbilden des Festlegungsbands nach einem der vorhergehenden Ansprüche, **gekennzeichnet durch** ein Ausbilden eines textilen Gewebes (14), das eine Schußfadenrichtung (12) aufweist, **durch** Wirken oder Weben, wobei das Gewebe Bänder bzw. Streifen aufweist, die sich in der Kettfadenrichtung (16) erstrecken, wobei wenigstens einer der Streifen (6) in der Schußfadenrichtung dehnbar ist und eines der Streifen (4 oder 8) nicht dehnbar ist, und **durch** ein Ausbilden des Bands aus einem Bereich (18) des Gewebes, wobei sich das Band in der Schußfadenrichtung des Gewebes erstreckt und Bereiche aus einem dehnbaren und einem nicht dehnbaren Streifen umfaßt.

14. Verfahren nach Anspruch 13, weiters **gekennzeichnet durch** ein Ausbilden eines Felds bzw. einer Anordnung von Festlegungselementen (20) in einem der nicht dehnbaren Streifen (4 oder 8).

15. Verfahren nach Anspruch 13, weiters **gekennzeichnet durch** ein Ausbilden eines Kettfadenmusters in den dehnbaren Streifen (6), das **durch** nicht elastische Kettfadengarne und elastische Kettfadengarne definiert wird, wobei die elastischen Kettfadengarne jeweils einen Bereich aufweisen, der sich in Schußfadenrichtung erstreckt, um wenigstens eines der nicht elastischen Kettfadengarne zu kreuzen.

16. Verfahren nach Anspruch 15, **dadurch gekennzeichnet, daß** die elastischen Kettfadengarne jeweils mehr als eines der nicht elastischen Kettfadengarne kreuzen.

17. Verfahren nach Anspruch 13, weiters **dadurch gekennzeichnet, daß** das Festlegungsband einen nicht dehnbaren Bereich an einem Gegenstand gesichert und einen über den Gegenstand vorragenden Bereich aufweist, wobei der vorragende Bereich bzw. Abschnitt einen dehnbaren Bereich und einen nicht dehnbaren Bereich aufweist, von welchem sich Festlegungselemente erstrecken, wobei der nicht dehnbare Bereich von dem Gegenstand durch den dehnbaren Bereich beabstandet ist.

## Revendications

1. Bande de fermeture (2) comprenant :
un support textile formé par une armure de fils sélectionnés définissant un sens des fils de chaîne et un sens des fils de trame, **caractérisée en ce que** le support textile (14), en raison de l'armure des fils sélectionnés, a, dans le sens des fils de trame (12), une première zone (4 ou 8) qui est sensiblement non extensible dans le sens des fils de trame et une seconde zone (6) qui est sensiblement extensible de manière élastique dans le sens des fils de trame.

2. Bande de fermeture selon la revendication 1, dans laquelle un ensemble d'éléments de fermeture (20) s'étend à partir de la première zone (4).

3. Bande de fermeture selon la revendication 1 ou la revendication 2, ayant des éléments de fermeture sous la forme de crochets s'engageant avec des crochets (20).

4. Bande de fermeture selon l'une quelconque des revendications précédentes, dans laquelle le support textile comprend au moins deux zones (4, 8) non extensibles dans le sens des fils de trame, l'une de ces zones disposant d'éléments de fermeture et l'une de ces zones n'ayant pas d'éléments de fermeture.

5. Bande de fermeture selon la revendication 4, dans laquelle l'une des zones non extensibles (4) dispose d'un ensemble d'éléments de fermeture et l'autre zone non extensible comprend une patte disposée de manière à être jointe à un article, par exemple une pièce de vêtement telle qu'une toile gaufrée.

6. Bande de fermeture selon l'une quelconque des revendications précédentes, dans laquelle la zone extensible de manière élastique comprend une armure en chaîne définie par des fils de chaîne non élastiques et des fils de chaîne élastiques, les fils de chaîne élastiques ayant chacun une partie s'étendant dans un sens des fils de trame pour croiser au moins l'un des fils de chaîne non élastiques.

7. Bande de fermeture selon la revendication 6, dans laquelle le support textile est tissé et comprend une armure gaze.

8. Bande de fermeture selon les revendications 6 ou 7, dans laquelle un grand nombre de fils parmi les fils de chaîne élastiques croisent chacun plus d'un fil de chaîne non élastique.

9. Bande de fermeture selon l'une quelconque des revendications 1 à 6, dans laquelle le support textile comprend un élément tricoté.

10. Bande de fermeture selon l'une quelconque des revendications précédentes, sous la forme d'une patte de fermeture ayant une partie fixée à un objet et une partie faisant saillie au-delà de l'objet, la partie en saillie ayant une zone extensible et une seconde zone à partir desquelles des éléments de fermeture s'étendent, la seconde zone étant séparée de l'objet par ladite zone extensible.

11. , Bande de fermeture selon la revendication 10, dans laquelle l'objet comprend un vêtement.

12. Bande de fermeture selon la revendication 11, dans laquelle le vêtement comprend une toile gaufrée.

13. Procédé de formation de la bande de fermeture selon l'une quelconque des revendications précédentes, **caractérisé par** la formation d'un tissu (14) ayant un sens des fils de trame (12) par tricotage ou par tissage, le tissu ayant des bandes s'étendant dans le sens des fils de chaîne (16), au moins l'une des bandes (6) étant extensible dans le sens des fils de trame, et l'une des bandes (4 ou 8) étant non extensible, et formant la bande à partir d'une partie (18) du tissu, la bande s'étendant dans le sens des fils de trame du tissu et comprenant des parties d'une bande extensible et non extensible.

14. Procédé selon la revendication 13, **caractérisé en outre par** la formation d'un ensemble d'éléments de fermeture (20) dans l'une desdites bandes non extensibles (4 ou 8).

15. Procédé selon la revendication 13, **caractérisé en outre par** la formation dans les bandes extensibles (6) d'une armure en chaîne définie par des fils de chaîne non élastiques et des fils de chaîne élastiques, les fils de chaîne élastiques ayant chacun une partie s'étendant dans le sens des fils de trame pour croiser au moins l'un des fils de chaîne non élastiques.

16. Procédé selon la revendication 15, **caractérisé en ce que** les fils de chaîne élastiques croisent chacun plus d'un fil de chaîne non élastique.

17. Procédé selon la revendication 13, **caractérisé en outre en ce que** la bande de. fermeture a une partie non extensible fixée à un objet et une partie faisant saillie au-delà de l'objet, la partie en saillie ayant une zone extensible et une zone non extensible à partir desquelles des éléments de fermeture s'étendent, la zone non extensible étant séparée de l'objet par la zone extensible.
